# EUROPEAN PATENT SPECIFICATION

(11) **EP 4 065 210 B1**
(45) Date of publication and mention of the grant of the patent: **01.01.2025**
(21) Application number: 20825167.8
(22) Date of filing: 27.11.2020
(51) Int. Cl.: A61N 1/05

(54) **NEURAL INTERFACE SYSTEM**
NEURALSCHNITTSTELLENSYSTEM
SYSTÈME D'INTERFACE NEURONALE

(30) Priority: 27.11.2019 US 201962941522 P
(43) Date of publication of application: 05.10.2022
(73) Proprietor: Galvani Bioelectronics Limited, Stevenage SG1 2NY (GB)
(72) Inventor: OUCHOUCHE, Sebastien, Brentford Middlesex TW8 9GS (GB); AU, Cindy, Brentford Middlesex TW8 9GS (GB); RYS, Kenneth Douglas, Brentford Middlesex TW8 9GS (GB); BURGE, Thomas, Brentford Middlesex TW8 9GS (GB)
(74) Representative: Carpmaels & Ransford LLP
(86) International application number: PCT/GB2020/053036
(87) International publication number: WO 2021/105699

(56) References cited:
- EP-B1- 2 903 685
- WO-A2-03/082080
- US-A1- 2011 093 034
- US-A1- 2014 094 888
- US-A1- 2014 228 923
- US-A1- 2019 261 875
- US-B2- 9 744 354

## Description

### TECHNICAL FIELD

The present invention relates generally to neural interface system, also referred to as a lead system, for use with a neuromodulation device configured to neuromodulate a target, and more particularly, to improving alignment of neural interface system.

### BACKGROUND

Neural interface system, or a lead system, implanted onto a target such as a nerve or a neurovascular bundle can provide electrical stimulation to nerves through one or more electrodes when used with a pulse generator. Neural interface system and nerve stimulation can vary widely, based on the application and intended effect of the device. Many neural interface systems benefit from a precise, and secure placement of electrodes onto a nerve bundle, and a proper fit of the neural interface system to the target for improved safety and efficacy of the system.

Improperly mounted or misaligned neural interface systems, particularly at a distal end of the neural interface system where the electrode is present, can lead to several undesirable consequences. For example, a gap between the electrode and targeted nerve can impact the effects of the neural interface system, leading to a loss of therapy, a need to deliver a significantly higher amount of current to achieve the same therapeutic effect, or reduced efficiency. Incorrect placement can also create higher pressure areas which can constrict and potentially permanently alter nearby vasculature. Other effects can include elevated inflammation at the site of implantation, increased fibrosis, heightened stimulation requirements, and in severe cases, nerve death.

Current methods to address and maintain proper neural interface system placement and create strain relief include coiling of lead bodies, e.g., loops or sigmoid shapes, during implantation. Coiling may be effective in early stages of implantation, but as fibrotic tissue forms around the coil junction, the loops become locked in place and lose their efficacy in providing strain relief. Fibrotic tissue often forms several weeks after implantation. In other devices, shapes may be formed into lead bodies made of polyurethane. A lead body may be provided between a proximal end of the neural interface system (where a connection to a pulse generator may be provided) and a distal end comprising electrodes. Thus, the lead body may comprise of electrical conductors for the electrodes. However, the polyurethane material is less biostable and less axially flexible than other materials, such as silicone.

Such designs are also typically inefficient for application on pulsating structures. These strain relief designs are not as effective once fibrotic tissue forms around the lead body, and "locks" the lead body into place, as discussed above. In addition, shapes and coils formed into the lead body of the neural interface system are remote from a distal end comprising the electrode, and as such, do not address the movement, e.g., vertical action, of the distal end (which may for example comprise a neural cuff) when implanted on pulsating structures. Micro-motions caused from pulsating arteries can cause a compromise to the neural interface's therapeutic performance and effectiveness.
US 2014/228923 A1 describes a neurostimulation lead that includes a lead body having a proximal portion and a distal portion and a first conductor extending through the lead body. An electrode cuff can be secured relative to the distal portion of the lead body. The electrode cuff includes a cuff body, a first tendril extending from a first region of the cuff body, a second tendril extending from a second region of the cuff body and a first electrode disposed on the cuff body and electrically connected to the first conductor.
EP 2903685 B1 describes a cuff electrode assembly for implantation on a target nerve. The cuff electrode assembly can include a resilient cuff body configured to be disposed about the target nerve. The cuff body includes a first end portion having a first free end, and a second end portion having a second free end. The cuff electrode assembly further includes a first arm member and a second arm member each projecting radially outward from the cuff body and spaced from one another along the cuff body. The cuff body can be configured such that a force applied to urge the first and second arm members toward one another defines an open configuration of the cuff body configured to allow the cuff body to be positioned around the target nerve. The cuff electrode assembly further includes an electrode oriented to provide electrical stimuli to the target nerve.
WO 03/082080 A2 describes devices, systems and methods by which the blood pressure, nervous system activity, and neurohormonal activity may be selectively and controllably reduced by activating baroreceptors. A baroreceptor activation device is positioned near a baroreceptor, preferably a baroreceptor located in the carotid sinus. A control system may be used to modulate the baroreceptor activation device. The control system may utilize an algorithm defining a stimulus regimen which promotes long term efficacy and reduces power requirements/consumption. The baroreceptor activation device may utilize electrodes to activate the baroreceptors. The electrodes may be adapted for connection to the carotid arteries at or near the carotid sinus, and may be designed to minimize extraneous tissue stimulation.
US 2011/093034 A1 describes an implantable medical lead that includes a proximal portion having first and second contacts. The lead further includes a first distal arm having a first electrode that is electrically coupled to the first contact, and includes a second distal arm having a second electrode that is electrically coupled to the second contact. The lead also includes a branch region where the proximal portion transitions to the first and second distal arms. A tissue anchoring element is attached to the branch region for securing the branch region to tissue of a patient into which the lead is implanted. Such bifurcated leads may be used to apply electrical signals to occipital nerves of the patient via the electrodes. A lead extension includes a distal connector with two lead receptacles and a tissue anchoring element attached to the connector. An adaptor having three lead receptacles includes an anchoring element attached thereto.
US 2019/261875 A1 describes stimulation and recording electrode assemblies that are particularly useful for Automatic Period Stimulation (APS). Such embodiments are compatible with nerve monitoring systems to provide continuous stimulation of a nerve during surgery. Certain embodiments include an electrode assembly having cuff including a body and two ears extending from the body. Within the body, at least one electrode is supported and connected to a lead wire assembly. The ears can be brought together to enlarge a gap in the body so that the electrode assembly can be fixated around a nerve. Other embodiments include an electrode assembly including first and second needle electrodes that each have a tip. A body is provided to interconnect the needle electrodes and can be manipulated to move the tips either toward or away from one another. Disclosed embodiments provide nerve monitoring and stimulation in cases where the nerve is only partially dissected.
US 2014/094888 A1 describes a cuff electrode assembly that includes a resilient cuff body configured to be disposed about a nerve. The cuff body includes a first end portion and a second end portion. The cuff body can be pre-formed to define a closed configuration having a generally annular cross-sectional shape such that, in the closed configuration, the cuff body extends helically with the first and second end portions overlapping on different planes. The cuff electrode assembly includes a first arm member and a second arm member, each projecting radially outward from the cuff body and spaced from one another along the cuff body. The cuff body can be configured such that force applied to urge the first and second arm members toward one another causes relative deflection of the first end portion and the second end portion to define an open configuration of the cuff body.
US 9 744 354 B2 describes devices, systems and methods of neurostimulation for treating obstructive sleep apnea.

### SUMMARY

The invention is defined in independent claim 1. Embodiments of the invention are defined in the dependent claims.

### BRIEF DESCRIPTION OF THE DRAWINGS

Various non-limiting embodiments are further described with reference to the accompanying drawings in which:
FIG. 1 depicts an example of a well-aligned cuff on a neurovascular bundle.
FIG. 2 depicts an example of an ill-aligned cuff on a neurovascular bundle.
FIG. 3 depicts a neural cuff in accordance with embodiments described herein.
FIG. 4 depicts a neural cuff having a hollowed spine.
FIG. 5 depicts a neural cuff having a plurality of strain relief notches.
FIGS. 6A and 6B depict variations for strain relief notches in accordance with embodiments described herein.
FIG. 7 depicts strain relief features on a spine of a neural cuff.
FIG. 8 depicts additional strain relief features in accordance with embodiments described herein.
FIG. 9A depicts a neural cuff having slanted arms.
FIG. 9B depicts a neural cuff having slanted electrode arrays.
FIGS. 10A and 10B depict neural cuffs having pivotable arms relative to the spine.
FIG. 11 depicts another design in which the cuff arms may be modified to accommodate various angles and positions within the target anatomy.
FIG. 12A is a line diagram of an electrode device and lead body according to an embodiment.
FIG. 12B is a line diagram of an electrode device and lead body according to another embodiment.

### DETAILED DESCRIPTION OF ILLUSTRATIVE EMBODIMENTS

Various aspects of the present disclosure described herein in are generally directed to devices, systems and methods for improving the security, efficacy and application of neural interface system, also referred to as a lead system, to a target, which may be a nerve or neurovascular bundles. It will be understood that the provided examples are solely for purposes of clarity and understanding and are not meant to limit or restrict the claimed subject matter or relevant portions of this disclosure in any manner. For example, in addition to applications of neural cuffs, which are described as an example of a distal end of the neural interface system comprising an electrode, other shapes or arrangements of distal end may be used, for example a neural patch. Further, such neural interface system may be applied to neurovascular bundles as well as any non-pulsating targets such as nerves.

In various embodiments described herein, a neural cuff may comprise one or more arms that are attached to a spine that provide mechanical stability and passage for wiring to electrodes. The arrangement of the neural cuff, including the shape or number of the one or more arms, spine, positioning of electrodes, and materials for each aspect may vary depending on one or more factors and considerations, such as flexibility, durability, and positioning considerations.

An example target may be a splenic neurovascular bundle, which is a complex of autonomic nerves wrapping around the splenic artery. A target implantation site may be a loop on the splenic artery that is sufficiently detached from the pancreas. However, the geometry of the splenic neurovascular bundle may be variable in humans. The curvature of the loop can differ between patients, and even within the same individual, due to a variety of factors, such as age and body mass index. As such, maintaining proper alignment of the neural interface system can be a challenge in some cases.

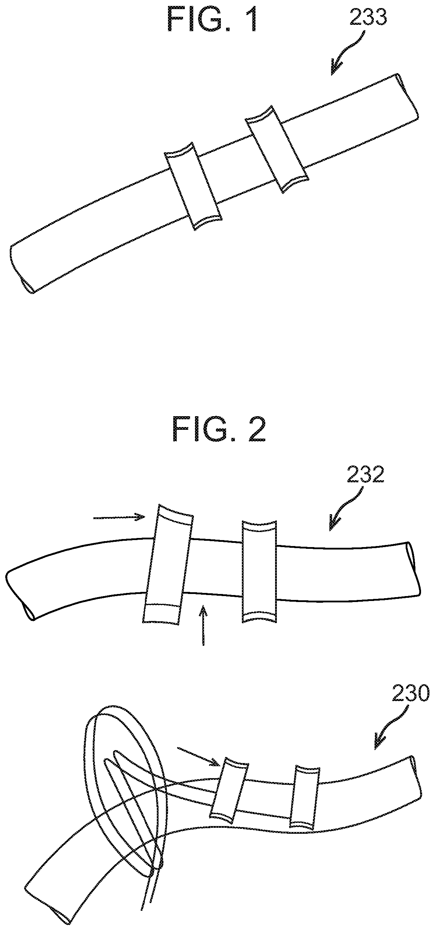

FIGS. 1-2 depict an example of neural interface system applied to a neurovascular bundle. The neural interface system shown in Figures 1-2 is a lead system comprising a neural cuff. Although neural interface system comprising a cuff portion is illustrated, it will be appreciated by the skilled person that the present disclosure applies to other neural interface systems comprising various other shapes and types of distal end. For example, the neural interface system may be paddle type, wrap-type or simply lead system mostly comprising of a lead body and a neural interface portion such as a cuff portion with exposed electrodes provided at various distal end portions of the lead body. FIG. 1 depicts an example of a well-aligned lead system 233, which is desired, while FIG. 2 illustrates two examples of ill-aligned lead systems 230 and 232.

One method to address the neurovascular bundle and splenic loop variation is manufacturing multiple arterial cuffs and neural interfaces to accommodate various curvatures. However, this method can require a plurality of designs, which may subsequently require additional efforts to determine that a correctly-sized cuff has been selected for a patient, prior to implantation. Therefore, one challenge is to accommodate for any changes in the morphology of the splenic artery within the patient, e.g., changes due to age.

Another challenge in designing a neural cuff is enabling the cuff system to conform to a pulsating artery, which can have a local, variable curvature and diameter, whilst minimizing pressure on the artery. One approach to address this challenge is using an electrode small enough to attach to a single portion of the artery. This approach can minimize pressure on the artery, however, the nerve coverage may be subpar and activate only a small percentage of the neurovascular bundle. Small electrodes may also require additional anchoring mechanisms, such as gluing or suturing, to remain in place. However, such securing methods introduce additional materials into the cuff system and implantation procedure, which must each be quality controlled and studied for biocompatibility and biostability. These methods can also make removal of the implant both difficult and risky, especially for sutures that are fragile and/or have a high-risk for hemorrhaging.

In addition to difficulties related to splenic loop geometry variation and potentially changing morphology (or even if the neural cuff is provided on a nerve or nerve bundle other than the splenic nerve), any chronically implanted neural cuffs are subjected to variable forces across patients due to differing levels and modes of activities, anatomical variation, and routing strategies. In chronic implantation, neural cuffs on dynamic pulsating biological structures, e.g., the splenic neurovascular bundle, are subject to forces that can shift and misplace the alignment of the neural interface system, particularly the distal end, on the neurovascular bundle over time.

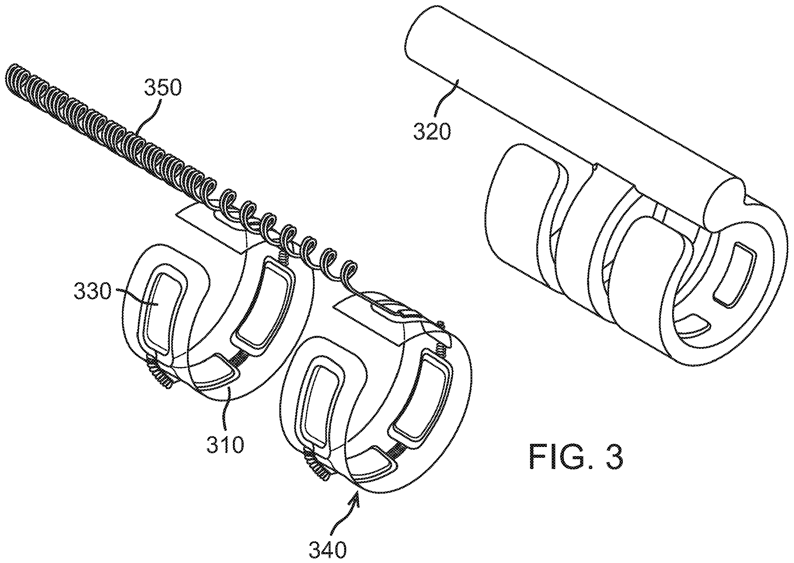

FIG. 3 illustrates an example of a neural interface system comprising a neural cuff at its distal end. As discussed above in relation to FIGS. 1 and 2, the present disclosure does not only apply to neural interface systems comprising a neural cuff, but also to other neural interface systems comprising various other shapes and types of the distal end. For example, the neural interface system may be paddle type, wrap-type or simply lead system mostly comprising of a lead body with exposed electrodes provided at various distal end portions of the lead body. In some embodiments, the neural interface system may be powered wirelessly by including a receiver or coil at the neural interface system instead of a lead body that provides a hard-wired connection. In some embodiments, the implantable pulse generator referred to herein need not be implanted, if the neural interface system can be powered by a wireless pulse generator such as a device worn by a user. In some other embodiments, the neural interface system may comprise a miniature implantable pulse generator (IPG) with wireless antenna for receiving power and communication from a transmitter. The IPG may receive power from an external source, and/or may comprise a battery for being charged from an external source, wherein the IPG is powered by said battery or an external source. While the following figures refer to wired lead-body based embodiments, it should be understood that these embodiments could alternatively be wireless, and that pulse generators described herein may be implanted although need not be implanted or implantable.

In accordance with some of the embodiments disclosed herein, that is formed through a two-shot molding process. The first shot 310 may comprise a stiffer durometer (e.g., at least Shore 70A) to prevent the electrode(s) from delamination, and the second shot 320 is significantly more flexible. In the second shot, the bulk of the cuff may have a significantly lower durometer than the first shot 310 in order to provide lower bending stiffness, and pressure asserted onto the target such as an artery when implanted. As a result, the neural cuff comprises a bi-layered design with differential durometers. In some examples, the cuff comprises silicone, and/or one or more other materials having biocompatibility and biostability qualities particular for the cuff's intended positioning and application. Likewise, the durometer of the first and second shot may vary.

In embodiments of the neural cuff, one or more electrodes 330 may be assembled onto cutout windows on the arms 340 of the first shot. The electrodes 330 provide contact with the neurovascular bundle when installed and are connected to a lead body conductor 350. In embodiments, the lead body conductor 350 may be welded to electrodes after the electrodes have been assembled onto the first shot 310. As will be evident in the various embodiments and examples discussed herein, the positioning of the electrodes on the arms, as well as the configurations and flexibility of the arms, spine, and attachment points can vary greatly depending on sizing, intended positioning, strain relief requirements, and potential expansion, movement, pulsation, and shape of the artery.

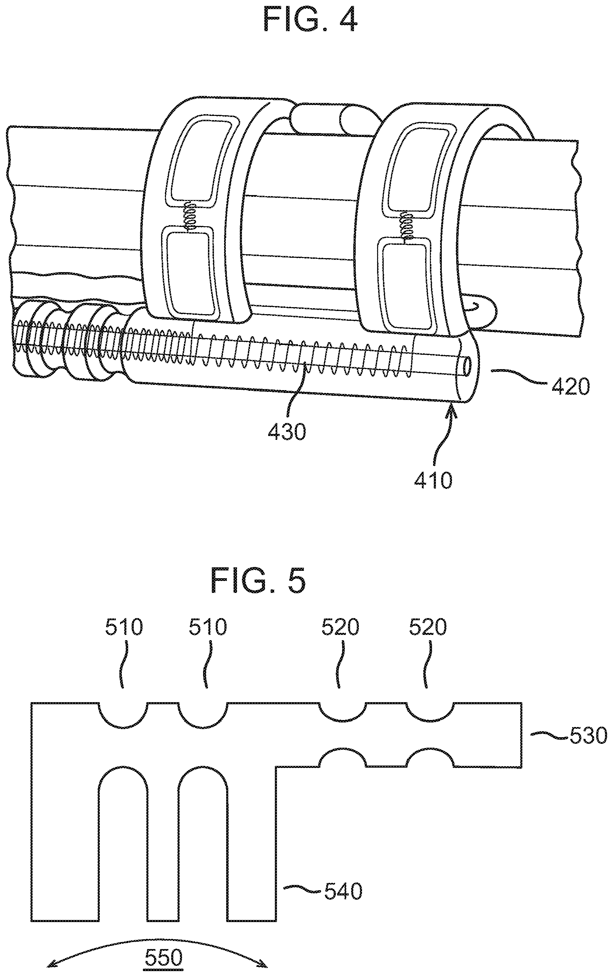

FIG. 4 depicts a neural cuff having a hollowed spine 410 which assists in lowering spine stiffness and increasing overall flexibility. In embodiments, the spine may be molded around a mandrel that is positioned through the main wiring coil 430 and removed after the molding has cured to create a central opening 420. The molding may occur, for example, in accordance with the two-shot molding process, discussed above. In other embodiments, the hollowed spine design may be achieved by forming a hole at a distal end after molding.

The hollowed spine design results may produce several advantages over conventional spine designs. First, the lower bending stiffness may allow the spine to conform to a curved structure. This may improve the cuff's application and stability to neurovascular bundles having varied shapes and sizes. In addition, the spinal flexibility may impart less pressure on the neurovascular bundles, given that the cuff can adapt to various curvatures. This may reduce issues and potential damage that can arise from excessive pressure and natural pulsation and movement of arteries.

The hollowed spine design may also provide advantages with respect to manufacturing. The mandrel may assist in preventing the wiring coil 430 from shifting during the molding process. The mandrel may assist in stabilizing the coil, and the design may help to prevent a bias causing a misalignment during the molding process, as well as undesirable pressure build-up, which can cause damage and reduce the effectiveness and lifespan of the neural cuff. In some embodiments, at least a part of the hollowed spine may be filled (or backfilled). For example, the hollowed spine may be filled with the material the spine or the other parts of the cuff is formed of, such as silicone or polyurethane. The hollowed spine may be filled at least partially, up to the point where the electrical conductors are provided. For example, referring to FIG. 4, the hollowed central opening 420 may be filled partially from the right edge shown on the figure (which can be referred to as a distal end of the spine, where a proximal end of the spine is connectable to a lead body or an extended spine portion) to a start of the coil conductor 430.

Similar advantages may be realized through one or more strain relief notches added to the spine of a neural cuff. FIG. 5 illustrates an example a cuff embodiment comprising a plurality of strain relief notches 510, 520 on the spine 530. In various examples, depending on desired spine flexibility and/or the contours of the neurovascular bundles, strain relief notches 510 may be added adjacent to, or between, the one or more arms 540. This may provide additional flexibility for movements of the arms 540. In another aspect, the notches 520 may be added to the lead body portion of the spine 530. These notches may aid in increasing the range of motion 550 of the lead body relative to the head. Again, the additional flexibility and range of motion of the spine can adapt to movement and various contours and curvatures of the neurovascular bundle, while also decreasing pressure on contact points. It will be appreciated that the depth of the strain relief notches may be determined or limited depending on the size of the spine lumen and the electrical conductor size or type provided in the spine lumen.

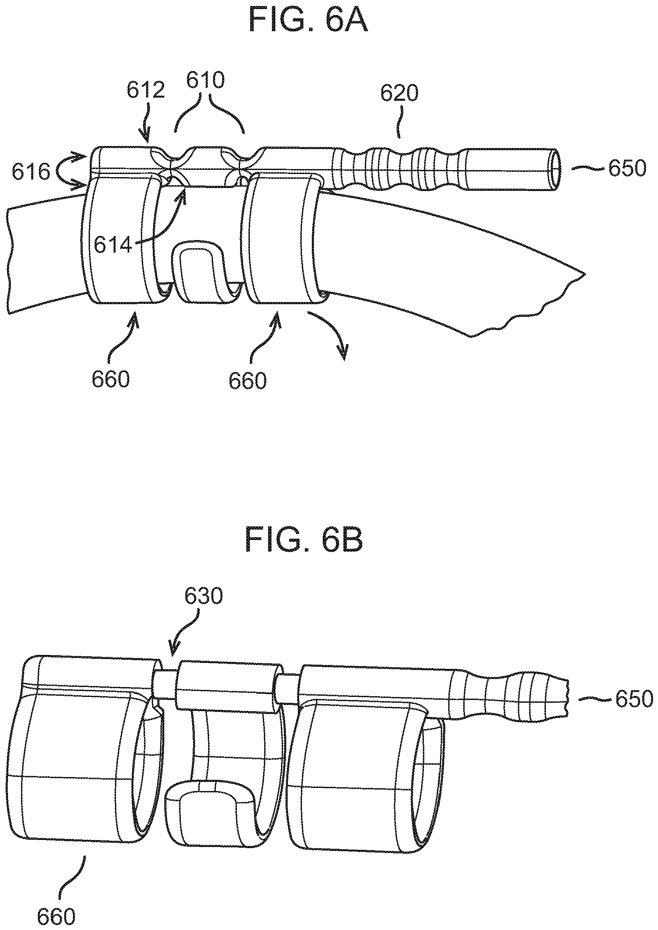

FIGS. 6A and 6B illustrate design variations of the strain relief notches. FIG. 6A illustrates two contour styles 610, 620 that partially or fully surround the circumference of the spine 650. Relief notches 610 may be located between each of the arms and provide flexibility to the arm portion relative to the lead body section of the spine. In particular, notches 610 may be limited to an upper side 612 of the spine (i.e., opposite the arms) and a lower side 614 of the spine, which may provide vertical flexibility 616 of the spine to accommodate curves and contours of the artery to which it may be attached. Since the notches 610 only partially surround the circumference of the spin 650, the spine may still maintain stiffness in other directions, e.g., horizontally. Strain relief notches 620 may comprise a different design having a circumferential cutout with a curved contour. This may provide additional flexibility for the lead body, particularly the spine portion, compared to notches 610. More specifically, the strain relief notches 620 may provide additional decoupling between the lead body and the cuff. Compared with straight circumferential cutouts, curved notches 620 may reduce stress on the spine during flexing. Strain relief notches 620 also assist in preventing additional pressure or stress on the wiring coil, as well as allowing for the cuff system to adapt to various movements and contours of the attached artery and neurovascular bodies. The strain relief notches 620 can provide a smoother stiffness gradient between the lead body and the cuff, which can improve flex fatigue performance of the neural interface system comprising the lead body and the cuff.

FIG. 6B depicts additional examples of notch designs on a neural cuff. Here, strain relief notches 630, located between arms 660, may comprise a vertical cutout that surrounds the circumference of spin 650. This style may provide greater flexibility to the arm portion of the cuff system, compared to notches 610 in FIG. 6A. The notches 640 on the lead body are similar to the contoured notches 620 in FIG. 6A, but with a more elongated, flatter contour. The contour design may reduce stiffness of the lead body portion of the spine and allow additional flexibility relative to the arm portion. In addition, although only 1-3 notches are depicted in each section of the spine 650 throughout FIGS. 6A-6B, any other number of notches may be implemented to accomplish desired flexibility and stiffness of the spine. For example, notches may comprise a threaded design, alone or in combination with any number of contour variations. As can be seen in FIG 6A, the target is curved, along a length of the neural interface system. In other words, there is a curvature in an axis of the target.

It will also be appreciated that the strain relief notch designs may encompass any of a variety of styles, designs, and variations, and are not limited to the examples depicted throughout the Figures. The depicted designs are for illustrative purposes only and various embodiments may include similar, different, or a combination of these and other designs, which are configured in accordance with embodiments described herein, to provide strain relief to the spine, lower spine stiffness, and increase spine flexibility.

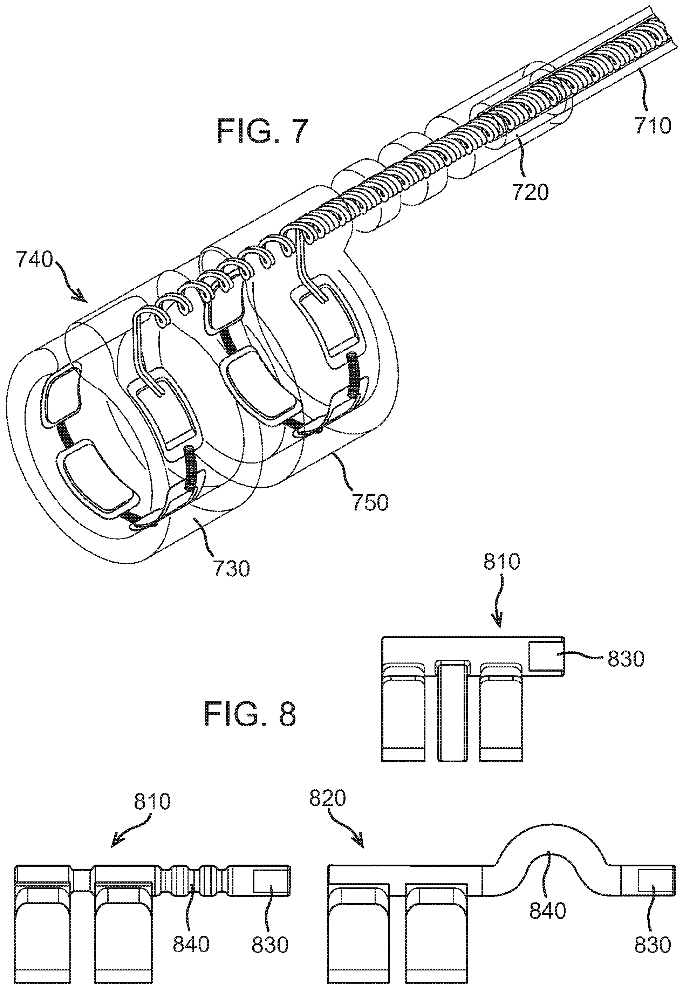

Turning to FIG. 7, an additional embodiment for providing strain relief to the neural cuff lead is depicted. In this embodiment, the lead body comprises an internal, thin-wall tubing 710 that surrounds the wire coil 720, or conductors, connected to the electrodes 730. In embodiments, the thin-wall tubing 710 may comprise silicone and have a thickness of 0.25 mm. The tubing 710 provides a layer of protection around the wire coil 720 (which in this case is a quadrifilar coradial conductor, where in other embodiments bi-filar co-radial conductor may be used for increased flexibility) and has a low spring constant for maximized or increased flexibility and stretchability. As such, the tubing may be able to adapt to (or in this case able to decouple) movement, pulsation, curves, and contours experienced by the cuff system. In embodiments, the thin-wall tubing 710 may be kept straight, to improve ease of handling and explanation procedures. For example, the straight tubing promotes a clean fibrotic channel, which eliminates or reduces issues and explantation difficulties that can be experienced by coiled and sigmoid tubing designs.

Similarly, the wire coil 720 itself may comprise a co-radial conductor having a low spring constant. Like the thin-wall tubing 710, the wire coil also has maximized or increased stretchability and flexibility to be able to adapt to movements, contours, and changes experienced by the cuff system.

The wire coil 720 and thin-wall tubing 710 may fit within the outer molding 740, which may comprise the elongated spine. As described in various embodiments herein, the spine may have one or more features for strain relief, such as one or more notches. The strain relief shaping allows the lead body to swivel around and pivot away from the one or more arms 750 and electrodes 730. The strain relief contours combined with the thin-wall tubing 710 may also decouple twisting motions experienced by the lead body from the cuff's mounting on a neurovascular bundle. In this manner, there may be reduced pressure on contact areas of the neurovascular bundle, as well as reduced force within the cuff system on the wire coil 720 during movements.

FIG. 8 illustrates even more strain relief features and compares such features to traditional neural cuff spines. In traditional cuff spines 810, there are no strain relief features. There is typically only a very short distance between the cuff and lead body, which results in forces being coupled between the lead body and the cuff. For example, a twisting motion on the lead body would transfer over to the cuff. If the cuff were installed on an artery, such twisting motions could cause damage and/or excessive pressure to the artery. In some instances, the electrodes may become misaligned and consequently, less effective, as a result of the unexpected twisting motion or other movement.

However, in the cuff and spine designs disclosed herein 820, one or more strain relief features may serve to elongate the distance between the cuff and the lead body junction 830. This greater distance, as well as the flexibility from the strain relief features may aid in decoupling forces between the cuff and lead body. As illustrated by strain relief features 840, molded pivot structures and sigmoid structures are several methods that may be used to lower spine stiffness, increase flexibility, de-couple forces between the cuff and lead body, and increase the longevity and effectiveness of the neural cuff.

In addition to one or more notches and strain relief features that may be implemented neural cuff systems, modifications to the cuff arms, including positioning of the attached electrodes may provide additional nerve coverage and stability, and improve the overall effectiveness of the neural cuff system.

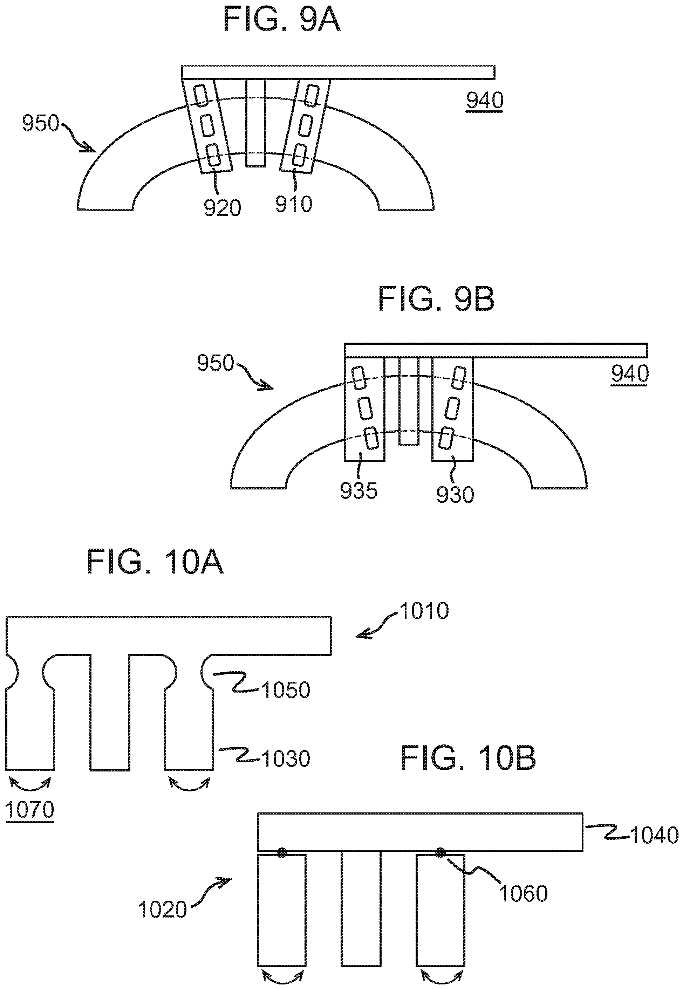

FIGS. 9A and 9B illustrate two examples that may be used to promote proper placement and stability of the one or more electrodes in their proper positions. Such designs may be particularly useful on neurovascular bundles having curves and contours, as depicted in the Figures. FIG. 9A illustrates a first embodiment wherein the arms 910 may be connected to the spine 940 at an angle. In traditional cuffs, the one or more arms may be positioned perpendicularly to the spine. Here, the arms may be positioned at an angle to maximize the proper placement of electrodes 920 on the curved target 950, such as an artery. In this example, the electrodes may be aligned vertically with respect to the cuff arm 910. As such, the placement of the electrodes 920 may follow the angle of the cuff arm 910 and can circumferentially engage the artery 950 at an optimal position. In various embodiments, the cuff arms 910 may be molded in the angled position through a molding process, e.g., the two-shot process, or any of a variety of methods.

FIG. 9B depicts a variation to FIG. 9A. In FIG. 9B, instead of the cuff arms being positioned at an angle relative to the cuff spine 940, the electrodes 935 may be positioned at the optimum angle for placement, while the one or more arms 930 are positioned perpendicularly to the cuff spine 940. In this embodiment, the design of the cuff system may be simpler, due to the traditional designs having perpendicular cuff arms relative to the spine. This design may also eliminate the preciseness required for determining an optimum arm angle, which may vary based on the morphology of the intended neurovascular bundle, and changes over time.

In various embodiments, the electrodes 935 may be movable within the cuff arms 930 to allow a precise angular placement relative to the artery 950. In other embodiments, the electrodes may be affixed to the cuff arm, as discussed with respect to FIG. 1. The electrodes 935 may be positioned at a predetermined angle, for example, to provide maximum contact and coverage to the artery, or an optimum angle determined for a particular neural cuff placement.

FIGS. 10A and 10B illustrate yet another variation of a cuff system design in accordance with one or more embodiments herein. In these examples, the one or more arms may pivot to provide additional flexibility 1070 relative the spine 1040. In FIG. 10A, one or more cutouts 1050 (e.g., notches, neckings, etc.) at or near the attachment point 1050 of the arm 1030 and spine 1040, in order to let the arms 1030 pivot. In FIG. 10B, the arm pivot is accomplished using one or more joints 1060, e.g., ball joints, applied between the arm 1030 and spine 1040. The joints may comprise any number of designs known in the art to allow the desired flexibility and movement between the spine and cuff arm.

The pivots may allow the arms to move in one or more directions, which can help ensure proper placement and stability of the electrodes. For example, pivotable arms allow the cuff system to be applied to neurovascular bundles and arteries comprising a plurality of shapes, curves, contours, and morphologies. Also, the pivotable arms can help the cuff system maintain its position when unexpected movements, forces, or other biological changes are applied to the cuff system.

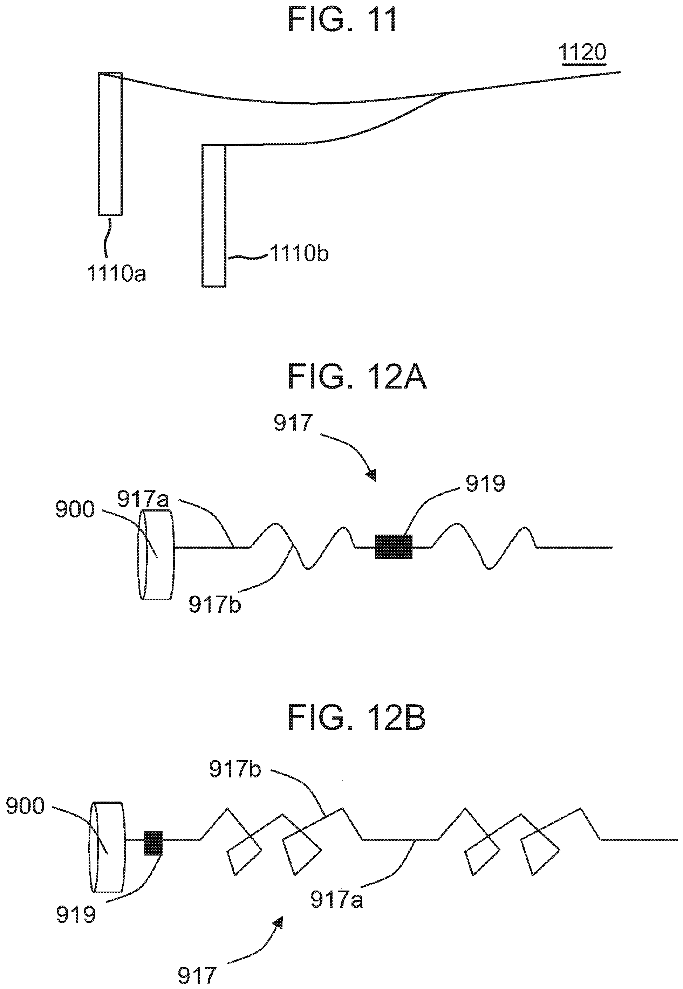

FIG. 11 depict another design in which the cuff arms may be modified to accommodate various angles and positions within the target anatomy. Instead of cuff arms being affixed to a spine as in other embodiments, the arms are de-coupled from the spine and lead body. Anode arm 1110a and cathode arm 1110b are not in a fixed position relative to each other, or even relative to a spine. As such, each arm 1110 may be precisely positioned and placed on the intended neurovascular bundle. This can provide significantly increased flexibility compared to both traditional cuff designs and other designs described herein. Such a design may almost completely de-couple forces between the arms, and significantly reduces any coupled forces between the lead body to the arms. Accordingly, the de-coupled arm design may easily accommodate various anatomical morphologies, curves, contours, and positions of arteries, and provides for increased flexibility and adaptability for implantation.

In other embodiments, one or both of lead body 650, 917 and conductor 350, 918 can comprise structures or configurations to provide strain relief. Referring also to FIGS. 12A and 12B, in some embodiments lead body 917 can comprise strain-relieving undulating sections 917b intermittently located between linear sections 917a. Any particular lead body 917 can comprise one undulating section 917b or a plurality of undulating sections 917b, and the particular configuration of the undulating section(s) 917b can vary. Undulating sections 917b help in breaking up or interrupting large or strong motions affecting lead body 917 into smaller, discrete or localized weaker movements.

Two examples of undulating sections 917b are depicted in FIGS. 12A and 12B, but these examples are not limiting with respect to all of the possible embodiments contemplated by this disclosure. For example, the undulations can be sinusoidal, square, rectangular, helical, coiled, regular, irregular, or other shapes or combinations of shapes. The number of undulations also can vary, with some undulating sections 917b having more or fewer undulations, as may be desired or preferred for areas that experience more or less strain in use. In general, however, each turn in an undulating pattern prevents pressure waves from migrating longer distances along the length of lead body 917.

In some embodiments, undulating sections 917b can be located near neural interface 900, while in other embodiments undulating sections 917b can be located away from neural interface 900 or at various points along the length of lead body 917. Undulating sections 917b near neural interface 900 can help to block displacement forces from reaching neural interface 900 and affecting its stability and placement.

The disclosed systems, methods, and devices may include neural cuffs comprising a plurality of electrodes; a spine providing a passage for conductors, also referred to as electrical conductors, to the plurality of electrodes; and at least two curved arms extending radially from a first portion the spine, wherein the plurality of electrodes are positioned on an inner circumference of the curved arms. In embodiments, the spine may comprise a plurality of strain relief notches positioned on the first portion, between the curved arms and on a second portion of the spine adjacent to the first portion and proximal to the curved arms, each of the plurality of notches partially or fully surrounding a circumference of the spine.

The strain relief notches may be positioned between the curved arms on a side of the spine opposite the attachment of the curved arms and provide flexibility to the first portion of the spine. In other embodiments, strain relief notches on the second portion of the spine fully surround the circumference of the spine and increase flexibility between the first portion and the second portion. Other variations of the strain relief notches include at least two notches on the second portion of the spine, cutouts that fully surround the circumference of the spine, and a length of notches on the second portion of the spine being greater than lengths of strain relief notches on the first portion.

In embodiments, the neural cuff may further comprise a tubing with a thickness up to 0.25 mm that surrounds the conductors and may be positioned within the spine. In other embodiments, the spine may be hollow, and may comprise silicone.

In various embodiments, curved arms may be pivotable relative to the spine. Various pivot designs, including one or notches at an attachment point between each of the curved arms and/or a ball joint at the attachment point may be used to allow each arm to pivot.

Additional cuff variations include (i) the curved arms extending perpendicular to the spine and the plurality of electrodes positioned on an inner circumference of the curved arms at an angle relative to the spine; and (ii) the curved arms fixed at an angle relative to the spine and the plurality of electrodes positioned vertically along the inner circumference of the curved arms.

A method for assembling the neural cuff includes providing a first shot comprising two or more curved arms; applying a plurality of electrodes to an inner circumference of the two or more curved arms; connecting a lead body conductor to the plurality of electrodes; providing a second shot to be an outer layer to the two or more curved arms and lead body, wherein the second shot has a lower durometer than the first shot; and molding the first shot to the second shot. Thus, the neural interface system may be provided by a shot molding process. The neural cuff (or neural interface system) can also be provided by various other methods including, for example, 3D printing and extrusion.

Systems methods, and device are disclosed herein for neural cuffs applied to neurovascular bundles. Various designs are provided herein to improve the attachment of the neural cuff and adapt to varying morphologies. In embodiments, the neural cuff comprises a plurality of electrodes, a spine providing a passage for electrical conductors to the electrodes, and two or more curved arms extending radially from the spine. The curved arms may be attached perpendicularly to the spine, or at an angle relative to the spine. Likewise, the electrodes, attached to an inner circumference of the curved arms, may be aligned or angled relative the curved arms. One or more strain relief notches may be applied to the spine to promote a proper placement of electrodes and to provide flexibility to the spine. Various embodiments may be assembled using a shot molding process.

Systems, methods, and devices are disclosed herein for improving neural cuffs applied to neurovascular bundles. In an embodiment, a neural cuff comprises a spine, a plurality of arms radially extending from the spine, and a plurality of electrodes positioned on an inner circumference on the curved arms. A plurality of strain relief notches positioned on the spine reduce spine stiffness and improve flexibility, which allows the neural cuffs to adapt to varying contours, movements, and morphologies of neurovascular bundles. The strain relief notches may be positioned on a first portion of the spine, between the curved arms, and on a second portion of the spine, adjacent to the first portion. The strain relief notches may fully or partially surround the circumference of the spine, and aid in providing increased flexibility between one or more portions of the spine, and between the curved arms. An implantable neural interface system comprises: at least one electrode; a spine comprising electrical conductors electrically connectable to a pulse generator and the at least one electrode; at least one arm extending from the spine, wherein the electrode is positioned on the arm; and a strain relief feature configured to reduce strain in relative movement or displacement of portions of the neural interface system.

An implantable neural interface system comprises: at least one electrode; a spine providing a passage for electrical conductors from an implantable pulse generator to the at least one electrode via a lead body; at least one arm extending from the spine, wherein the electrode is positioned on the arm; and a strain relief feature configured to decouple movement between the lead body and the spine.

An implantable neural interface system comprises: at least one electrode; a spine providing a passage for electrical conductors from an implantable pulse generator to the at least one electrode via a lead body; at least one arm extending from the spine, wherein the electrode is positioned on the arm; and a strain relief feature configured to decouple movement between the lead body and the arm.

An implantable neural interface system comprises: at least one electrode; a spine for electrical conductors for the at least one electrode; at least one arm extending from the spine, wherein the electrode is positioned on the arm; and a strain relief feature.

An implantable neural interface system comprises: at least one electrode; a spine providing a passage for electrical conductors from an implantable pulse generator to the at least one electrode via a lead body; at least one arm extending from the spine, wherein the electrode is positioned on the arm; and a strain relief feature.

An implantable neural interface system comprises: at least one electrode; a spine providing a passage for electrical conductors from an implantable pulse generator to the at least one electrode; at least one arm extending from the spine, wherein the electrode is positioned on the arm; and a strain relief feature configured to reduce strain in relative movement of one or more of the spine and the at least one arm to accommodate a curvature in an axis of a target on or in which the spine and at least one arm are provided.

An implantable neural interface system comprises: at least one electrode; a spine providing a passage for electrical conductors from an implantable pulse generator to the at least one electrode; at least one arm extending from the spine, wherein the electrode is positioned on the arm; and a strain relief feature configured to reduce strain in relative movement or displacement of portions of neural interface system to accommodate a curvature in an axis of a target on or in which the neural interface is provided.

The strain relief feature provides reduced strain compared to a portion in which the strain relief feature is not provided.

The strain relief feature may provide a curvature in an axis of the neural interface system, wherein the axis of the neural interface is, or is parallel to, an axis of the spine. In other words, the target, which often comprises a generally tubular shape may comprise a curved axis. These strain relief features may help accommodate such curvature in the curved axis of the target.

The strain relief feature may comprise at least one of: a notch, a j oint, a ball j oint, a portion comprising higher flexibility material than its surrounding portion, and a reduced cross-sectional area. Such reduced cross-sectional area may be achieved in various ways, including providing notches which fully surrounds a circumference of the spine or notches which only partially surrounds a circumference of the spine. The reduced cross-section area may be achieved by providing at least partly hollow portion or portions in the spine.

The strain relief feature may: increase flexibility for relative movement of portions of the neural interface system; and/or enable relative movement of portions of the neural interface system, optionally wherein the movement is in a perpendicular direction to the axis of the target, optionally wherein the movement results in bending of the neural interface such that there is a curvature in a length of the neural interface parallel to the axis of the target.

The strain relief feature may be provided at an attachment region between the at least one arm which is curved and the spine thereby the at least one arm is pivotable at an angle relative to an axis of the spine.

The neural interface system may comprise a plurality of arms, wherein the spine comprises the strain relief feature positioned between the curved arms and a portion of the spine proximal to the curved arms, and each of the strain relief features partially or fully surround a circumference of the spine.

The neural interface system may comprise a silicone tubing surrounding the electrical conductors positioned inside the spine.

The neural interface system may comprise an extended spine portion between the spine portion from which the arms extend and a lead body comprising a part of the conductor between the spine and an implantable pulse generator, wherein the strain relief feature is provided in the extended spine portion. For example, such extended spine portion is shown in FIG 6A where a strain relief feature 620 is provided in this extended spine portion.

At least two curved arms may at least partially extend radially from a first portion the spine, and the spine may comprise the strain relief feature positioned on the first portion, between the curved arms and on a second portion of the spine adjacent to the first portion and proximal to the curved arms. The second portion may also be referred to as an extended spine portion.

The strain relief feature may be provided between the arms furthest from a lead body.

The neural interface system may comprise a tubing surrounding the conductors and positioned inside the spine.

The tubing may have a thickness up to 0.25 mm.

The spine may be hollow at least in part.

The spine may comprise silicone or polyurethane.

The strain relief feature between the curved arms may be positioned on a side of the spine opposite the attachment of the curved arms and provide flexibility to the first portion of the spine.

The strain relief feature on the second portion of the spine may fully surround the circumference of the spine and increase flexibility between the first portion and the second portion.

There may be at least two strain relief features on the second portion of the spine.

The strain relief feature may comprise a cutout that fully surrounds the circumference of the spine.

The neural interface system as described above, wherein a length of the strain relief feature on the second portion of the spine are greater than a length of the strain relief feature on the first portion.

A two-shot molding process may be used to radially attach the arms to the spine.

A method may comprise: connecting conductors through a body of a spine to a plurality of electrodes; radially attaching two or more curved arms to a first portion of a spine, wherein the plurality of electrodes are positioned on an inner circumference of the curved arms; and providing a plurality of strain relief feature on the first portion of the spine between the curved arms, and on a second portion of the spine adjacent to the first portion, wherein each of the strain relief feature partially or fully surround the circumference of the spine.

A two-shot molding process may be used to radially attach the curved arms to the spine.

A neural interface system may comprise: a plurality of electrodes; a spine providing a passage for conductors to the plurality of electrodes; and two or more curved arms extending radially from the spine; wherein (i) the curved arms extend perpendicular to the spine and the plurality of electrodes are positioned on an inner circumference of the curved arms, at an angle relative to the spine; or (ii) the curved arms are fixed at an angle relative to the spine and the plurality of electrodes are positioned vertically along the inner circumference of the curved arms.

The spine may comprise a plurality of strain relief feature positioned between the curved arms and a portion of the spine proximal to the curved arms, and each of the strain relief feature may partially or fully surround the circumference of the spine.

The arms may be formed by a first shot and an outer layer to the arms and the lead body are formed by a second shot which overmolds the arms.

The second shot may have a lower hardness than the first shot.

In other embodiments, the second shot may have a higher hardness than the first shot in some embodiments. The second shot may be formed of a conductive silicone. The electrodes may be formed of a conductive silicone.

The first shot may have a durometer of at least Shore 70A.

The second shot may comprise silicone.

Different materials may be used for the first and second shots.

A method for manufacturing a neural cuff may comprise: providing a first shot comprising two or more curved arms; applying a plurality of electrodes to an inner circumference of the two or more curved arms; connecting a lead body conductor to the plurality of electrodes; providing a second shot to be an outer layer to the two or more curved arms and lead body, wherein the second shot has a lower durometer than the first shot; and molding the first shot to the second shot.

The two or more curved arms may be positioned at an angle relative to the second shot.

The plurality of electrodes may be positioned at an angle on each curved arm.

Each of the curved arms may be in a fixed position relative to the spine when provided at an angle or when the electrodes are positioned at an angle.

A neural interface system may comprise a proximal end (the lead connector), a lead body comprising conductors (for example, coil and cables) and incluators (e.g. silicone tubing, PU tubing), and a distal end comprising a substrate (e.g. the cuff portion) and electrodes (or an array of electrodes).

The lead body may comprise increased flexibility in a portion closer to the cuff portion compared to a portion of the lead body further away from the cuff portion.

Systems and device are disclosed herein for neural cuffs applied to neurovascular bundles. Various designs are provided herein to improve the attachment of the neural cuff and adapt to varying morphologies. In embodiments, the neural cuff comprises a plurality of electrodes, a spine providing a passage for leads to the electrodes, and two or more curved arms extending radially from the spine. The curved arms may be attached perpendicularly to the spine, or at an angle relative to the spine. Likewise, the electrodes, attached to an inner circumference of the curved arms, may be aligned or angled relative the curved arms. One or more strain relief notches may be applied to the spine to promote a proper placement of electrodes and provide flexibility to the spine. Various embodiments may be assembled using a shot molding process.

Systems, methods, and devices are disclosed herein for improving neural cuffs applied to neurovascular bundles. In an embodiment, a neural cuff comprises a spine, a plurality of arms radially extending from the spine, and a plurality of electrodes positioned on an inner circumference on the curved arms. A plurality of strain relief notches positioned on the spine may reduce spine stiffness and improve flexibility, which allows the neural cuffs to adapt to varying contours, movements, and morphologies of neurovascular bundles. The strain relief notches may be positioned on a first portion of the spine, between the curved arms, and on a second portion of the spine, adjacent to the first portion. The strain relief notches may fully or partially surround the circumference of the spine, and aid in providing increased flexibility between one or more portions of the spine, and between the curved arms.

It will be appreciated that the various features and processes described above may be used independently of one another, or may be combined in various ways. All possible combinations and sub-combinations are intended to fall within the scope of this disclosure.

Conditional language used herein, such as, among others, "can," "could," "might," "may," "e.g.," and the like, unless specifically stated otherwise, or otherwise understood within the context as used, is generally intended to convey that certain embodiments include, while other embodiments do not include, certain features, elements, and/or steps. Thus, such conditional language is not generally intended to imply that features, elements and/or steps are in any way required for one or more embodiments or that one or more embodiments necessarily include logic for deciding, with or without author input or prompting, whether these features, elements and/or steps are included or are to be performed in any particular embodiment. The terms "comprising," "including," "having," and the like are synonymous and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list.

## Claims

1. An implantable neural interface system, comprising:
at least one electrode (330);
a spine (410), optionally wherein the spine (410) is configured to provide a passage for electrical conductors from an implantable pulse generator to the at least one electrode (330); and
at least one arm (340) extending from the spine (410), wherein the electrode (330) is positioned on the arm (340);
a strain relief feature (610, 620) configured to reduce strain in relative movement or displacement of portions of neural interface system,
optionally wherein the strain relief feature (610, 620) is configured to reduce strain in relative movement of one or more of the spine (410) and the at least one arm (340);
**characterized in that**
the at least one arm (340) comprises a first shot portion (310) and a second shot outer layer (320), and wherein the second shot outer layer (320) has a lower hardness than the first shot portion (310).

2. The neural interface system of claim 1, wherein the strain relief feature (610, 620) comprises at least one of: a notch, a joint, a ball joint, a portion comprising higher flexibility material than its surrounding portion, and a reduced cross-sectional area.

3. The neural interface system of any preceding claim, wherein the strain relief feature (610) is provided at an attachment region between the at least one arm (340) which is curved and the spine (410) thereby the at least one arm (340) is pivotable at an angle relative to an axis of the spine (410).

4. The neural interface system of any preceding claim wherein the at least one arm comprises a plurality of curved arms, wherein the spine (410) comprises the strain relief feature (610) positioned between the curved arms and a portion of the spine (410) proximal to the curved arms.

5. The neural interface system of claim 4, wherein at least two curved arms at least partially extend radially from a first portion the spine (410), and wherein the spine (410) comprises the strain relief feature (610, 620) positioned on the first portion, between the curved arms and on a second portion of the spine adjacent to the first portion and proximal to the curved arms.

6. The neural interface system of claim 4 or 5, further comprising a lead body, wherein the strain relief feature (610) is provided between the arms furthest from the lead body.

7. The neural interface system of any preceding claim, further comprising a lead body, wherein the lead body comprises electrical conductors (720) and a tubing (710) surrounding the electrical conductors (720), wherein the tubing is positioned inside the spine (410),
optionally wherein the tubing (710) has a thickness up to 0.25 mm,
optionally wherein the tubing (710) is a silicone tubing.

8. The neural interface system of any preceding claim, wherein the spine (410) is hollow, and/or wherein the spine (410) comprises silicone or polyurethane.

9. The neural interface system of claim 5 wherein the strain relief feature (610) between the curved arms are positioned on a side of the spine (410) opposite the attachment of the curved arms and provide flexibility to the first portion of the spine (410).

10. The neural interface system of claim 5, wherein there are at least two strain relief features (620) on the second portion of the spine (410).

11. The neural interface system of claim 5 or claim 10 wherein a length of the strain relief (620) feature on the second portion of the spine are greater than a length of the strain relief feature (610) on the first portion.

12. The neural interface system of any preceding claim , wherein the first shot portion (310) has a durometer of at least Shore 70A,
And/or wherein the second shot outer layer (320) comprises silicone,
And/or wherein different materials are used for the first shot portion (310) and second (320) shot outer layer.

## Patentansprüche

1. Implantierbares Neuralschnittstellensystem, umfassend:
mindestens eine Elektrode (330);
ein Rückgrat (410), wobei das Rückgrat (410) gegebenenfalls ausgestaltet ist, um einen Durchgang für elektrische Leiter von einem implantierbaren Pulsgenerator zu der mindestens einen Elektrode (330) bereitzustellen; und
mindestens einen Arm (340), der sich von dem Rückgrat (410) erstreckt, wobei die Elektrode (330) auf dem Arm (340) positioniert ist;
ein Zugentlastungsmerkmal (610, 620), das ausgestaltet ist, um Zug in relativer Bewegung oder Verschiebung von Anteilen des Neuralschnittstellensystems zu reduzieren, wobei das Zugentlastungsmerkmal (610, 620) gegebenenfalls ausgestaltet ist, um Zug in relativer Bewegung von einem oder mehreren von dem Rückgrat (410) und dem mindestens einen Arm (340) zu reduzieren;
**dadurch gekennzeichnet, dass**
der mindestens eine Arm (340) einen ersten Schussanteil (310) und eine zweite Schussaußenschicht (320) umfasst, und wobei die zweite Schussaußenschicht (320) eine geringere Härte als der erste Schussanteil (310) aufweist.

2. Neuralschnittstellensystem nach Anspruch 1, wobei das Zugentlastungsmerkmal (610, 620) mindestens eines von einer Kerbe, einem Gelenk, einem Kugelgelenk, einem Anteil, der Material mit höherer Flexibilität als sein Umgebungsanteil umfasst, und einem Bereich mit reduziertem Querschnitt umfasst.

3. Neuralschnittstellensystem nach einem der vorhergehenden Ansprüche, wobei das Zugentlastungsmerkmal (610) an einer Befestigungsregion zwischen dem mindestens einem Arm (340), der gekrümmt ist, und dem Rückgrat (410) bereitgestellt wird, wodurch der mindestens eine Arm (340) in einem Winkel relativ zu einer Achse des Rückgrats (410) schwenkbar ist.

4. Neuralschnittstellensystem nach einem der vorhergehenden Ansprüche, wobei der mindestens eine Arm eine Vielzahl von gekrümmten Armen umfasst, wobei das Rückgrat (410) das Zugentlastungsmerkmal (610) umfasst, das zwischen den gekrümmten Armen und einem Anteil des Rückgrats (410) proximal zu den gekrümmten Armen positioniert ist.

5. Neuralschnittstellensystem nach Anspruch 4, wobei mindestens zwei gekrümmte Arme sich mindestens teilweise radial von einem ersten Anteil des Rückgrats (410) erstrecken, und wobei das Rückgrat (410) das Zugentlastungsmerkmal (610, 620) umfasst, das auf dem ersten Anteil zwischen den gekrümmten Armen und auf einem zweiten Anteil des Rückgrats benachbart zu dem ersten Anteil und proximal zu den gekrümmten Armen positioniert ist.

6. Neuralschnittstellensystem nach Anspruch 4 oder 5, des Weiteren umfassend einen Zuleitungskörper, wobei das Zugentlastungsmerkmal (610) zwischen den Armen am weitesten entfernt von dem Zuleitungskörper bereitgestellt wird.

7. Neuralschnittstellensystem nach einem der vorhergehenden Ansprüche, des Weiteren umfassend einen Zuleitungskörper, wobei der Zuleitungskörper elektrische Leiter (720) und einen Schlauch (710) umfasst, welcher die elektrischen Leiter (720) umgibt, wobei der Schlauch innerhalb des Rückgrats (410) angeordnet ist, wobei der Schlauch (710) gegebenenfalls eine Dicke bis zu 0,25 mm aufweist,
wobei der Schlauch (710) gegebenenfalls ein Silikonschlauch ist.

8. Neuralschnittstellensystem nach einem der vorhergehenden Ansprüche, wobei das Rückgrat (410) hohl ist, und/oder wobei das Rückgrat (410) Silikon oder Polyurethan umfasst.

9. Neuralschnittstellensystem nach Anspruch 5, wobei das Zugentlastungsmerkmal (610) zwischen den gekrümmten Armen auf einer Seite des Rückgrats (410) gegenüber der Befestigung der gekrümmten Arme positioniert ist und dem ersten Anteil des Rückgrats (410) Flexibilität bereitstellt.

10. Neuralschnittstellensystem nach Anspruch 5, wobei es mindestens zwei Zugentlastungsmerkmale (620) auf dem zweiten Anteil des Rückgrats (410) gibt.

11. Neuralschnittstellensystem nach Anspruch 5 oder Anspruch 10, wobei eine Länge des Zugentlastungsmerkmals (620) auf dem zweiten Anteil des Rückgrats größer als eine Länge des Zugentlastungsmerkmals (610) auf dem ersten Anteil ist.

12. Neuralschnittstellensystem nach einem der vorhergehenden Ansprüche, wobei der erste Schussanteil (310) eine Durometer-Härte von mindestens Shore 70A aufweist,
und/oder wobei die zweite Schussaußenschicht (320) Silikon umfasst,
und/oder wobei für den ersten Schussanteil (310) und die zweite (320) Schussaußenschicht unterschiedliche Materialien verwendet werden.

## Revendications

1. Système d'interface neurale implantable comprenant :
au moins une électrode (330) ;
une colonne vertébrale (410), éventuellement la colonne vertébrale (410) étant configurée pour fournir un passage pour les conducteurs électriques d'un générateur d'impulsions implantable à l'au moins une électrode (330) ; et
au moins un bras (340) s'étendant à partir de la colonne vertébrale (410), l'électrode (330) étant positionnée sur le bras (340) ;
un élément de soulagement de contrainte (610, 620) configuré pour réduire la contrainte dans le mouvement relatif ou le déplacement de parties du système d'interface neurale,
éventuellement, l'élément de soulagement de contrainte (610, 620) étant configuré pour réduire la contrainte dans le mouvement relatif d'un ou de plusieurs de la colonne vertébrale (410) et de l'au moins un bras (340) ;
**caractérisé en ce que** l'au moins un bras (340) comprend une première partie tirée (310) et une deuxième couche extérieure tirée (320), et **en ce que** la deuxième couche extérieure tirée (320) a une dureté inférieure à celle de la première partie tirée (310).

2. Système d'interface neurale selon la revendication 1, l'élément de soulagement de contrainte (610, 620) comprenant au moins l'un des éléments suivants : une encoche, une articulation, une rotule, une partie comprenant un matériau plus flexible que sa partie environnante et une zone de section transversale réduite.

3. Système d'interface neurale selon n'importe quelle revendication précédente, l'élément de soulagement de contrainte (610) étant prévu dans une région de fixation entre l'au moins un bras (340) qui est courbé et la colonne vertébrale (410), de sorte que l'au moins un bras (340) peut pivoter selon un angle par rapport à un axe de la colonne vertébrale (410).

4. Système d'interface neurale selon n'importe quelle revendication précédente, l'au moins un bras comprenant une pluralité de bras incurvés, la colonne vertébrale (410) comprenant l'élément de soulagement de contrainte (610) positionné entre les bras incurvés et une partie de la colonne vertébrale (410) proximale aux bras incurvés.

5. Système d'interface neurale selon la revendication 4, au moins deux bras incurvés s'étendant au moins partiellement radialement à partir d'une première partie de la colonne vertébrale (410), et la colonne vertébrale (410) comprenant l'élément de soulagement de contrainte (610, 620) positionné sur la première partie, entre les bras incurvés et sur une deuxième partie de la colonne vertébrale adjacente à la première partie et proximale par rapport aux bras incurvés.

6. Système d'interface neurale selon la revendication 4 ou 5, comprenant en outre un corps de cordon, l'élément de soulagement de contrainte (610) étant fourni entre les bras les plus éloignés du corps de cordon.

7. Système d'interface neurale selon n'importe quelle revendication précédente, comprenant en outre un corps de cordon, le corps de cordon comprenant des conducteurs électriques (720) et une tubulure (710) entourant les conducteurs électriques (720), la tubulure étant positionnée à l'intérieur de la colonne vertébrale (410),
éventuellement, la tubulure (710) ayant une épaisseur allant jusqu'à 0,25 mm,
éventuellement, la tubulure (710) étant une tubulure en silicone.

8. Système d'interface neurale selon n'importe quelle revendication précédente, la colonne vertébrale (410) étant creuse, et/ou la colonne vertébrale (410) comprenant du silicone ou du polyuréthane.

9. Système d'interface neurale selon la revendication 5, l'élément de soulagement de contrainte (610) entre les bras incurvés étant positionné sur un côté de la colonne vertébrale (410) opposé à la fixation des bras incurvés et fournissant une flexibilité à la première partie de la colonne vertébrale (410).

10. Système d'interface neurale selon la revendication 5, au moins deux éléments de soulagement de contrainte (620) se trouvant sur la deuxième partie de la colonne vertébrale (410).

11. Système d'interface neurale selon la revendication 5 ou selon la revendication 10, une longueur de l'élément de soulagement de contrainte (620) sur la deuxième partie de la colonne vertébrale étant supérieure à une longueur de l'élément de soulagement de contrainte (610) sur la première partie.

12. Système d'interface neurale selon n'importe quelle revendication précédente, la première partie tirée (310) ayant un duromètre d'au moins Shore 70A,
et/ou la seconde couche extérieure tirée (320) comprenant du silicone,
et/ou des matériaux différents étant utilisés pour la première partie tirée (310) et la seconde (320) couche extérieure tirée.
